# EUROPEAN PATENT APPLICATION

(11) **EP 4 813 389 A1**
(43) Date of publication of application: **30.09.2026**
(21) Application number: 25166037.9
(22) Date of filing: 25.03.2025
(51) Int. Cl.: A61K 31/495, A61K 31/685, A61P 35/00

(54) **EDELFOSINE FOR USE IN THE TREATMENT OF GLIOMAS**

(71) Applicant: RDP Pharma AG, 8590 Romanshorn (CH)
(72) Inventor: The designation of the inventor has not yet been filed
(74) Representative: Forstmeyer, Dietmar

(57) **Abstract**

The present invention relates to the use of the alkyl phospholipid edelfosine (especially (S)-edelfosine) in the treatment of gliomas.

## Description

The present invention relates to the use of the alkyl phospholipid edelfosine (CP201; especially (S)-edelfosine, S-CP201) in the treatment of gliomas.

Gliomas are a diverse group of tumors originating from glial cells in the brain and spinal cord, representing the most common primary brain tumor within the central nervous system (CNS). In the United States, there are six cases of gliomas diagnosed per 100,000 individuals every year. Gliomas can be well-circumscribed or diffusely infiltrative tumors that affect all areas of the brain (Mesfin et al, 2024).

In the past, histologic diagnosis of gliomas provided a valuable foundation for assessing prognosis and guiding therapeutic management. They were classified based on their phenotypic cell characteristics (e.g. astrocytomas, ependymomas, and oligodendrogliomas). More recently, according to the WHO 2021 recommendations, molecular markers (e.g., isocitrate-dehydrogenase [IDH] and 1p/19q co-deletion) are used to categorize gliomas. For instance, astrocytomas are now diagnosed solely by IDH mutations. Oligodendrogliomas, by definition, have IDH mutations and 1p/19q co-deletions. Glioblastomas are now only IDH-wildtype tumors and no longer classified as IDH-mutant (Mesfin et al, 2024; Chen et al, 2024).

There are an estimated 80,000 to 90,000 newly diagnosed cases of primary brain tumors each year in the United States, with approximately 25% being gliomas. The total number of glioblastoma cases diagnosed each year is about 12,000 (approximately 15% of the total of newly diagnosed brain tumors and roughly 50% of all malignant brain tumors). Gliomas are most often present in patients without any relevant history (Mesfin et al, 2024).

Most patients are symptomatic at diagnosis, suffering from headaches, seizures, nausea, vomiting, and alike.

Imaging diagnostics are appropriate for evaluating suspected brain tumors, including computed tomography (CT) of the head to check for primary tumors as well as chest, abdomen, and pelvis for metastases; magnetic resonance imaging (MRI) of the brain and the spine; diffusion tensor imaging of the brain; and functional magnetic resonance imaging (Mesfin et al, 2024).

Low-grade gliomas (WHO grade I and II) generally have a more favorable prognosis with longer survival rates, particularly when complete surgical resection is achievable. High-grade gliomas (WHO grade III and IV), including glioblastomas, have a much poorer prognosis due to their aggressive nature and high propensity for recurrence. As mentioned, advances in molecular profiling have led to more precise prognostic information, identifying markers such as IDH mutations and 1p/19q co-deletions that are associated with better outcomes. Despite these advancements, the overall survival for high-grade gliomas remains limited, highlighting the urgent need for continued research and development of new therapeutic strategies (Schaff and Mellinghoff, 2024).

Gliomas, particularly high-grade forms like glioblastomas, pose numerous complications that significantly impact patient outcomes and quality of life. These complications arise from the tumor's infiltrative nature, which can lead to extensive neurological deficits. Additionally, treatment-related adverse effects further complicate management and recovery.

The most important treatment modality of gliomas is surgical resection, with goal of maximal safe resection, depending on tumor grading and location. In some cases (e.g., high grade gliomas, such as anaplastic astrocytomas and glioblastomas, radiation or radiation in combination with radiotherapy (radiochemotherapy) are used. Gliomas are also treated with chemotherapeutic agents, but only a limited number of agents are approved. The most common licensed cytotoxic drug is the DNA-alkylating agent temozolomide to treat for instance glioblastoma multiforme or astrocytoma. Older alkylating drugs used as anti-glioma drugs are lomustine or carmustine. The PVC regimen (procarbazine, lomustine, and vincristine) is recommended by medical guidelines to treat certain types of progressive IDH-mutant astrocytomas. Bevacizumab (monoclonal antibody directed against VEGF) is approved for treatment of recurrent glioblastoma. Nitrosourea may be also used for certain advanced gliomas. More recently, vorasidenib citrate (inhibitor of mutant isocitrate dehydrogenase to treat Grade 2 IDH-mutant glioma) was introduced for treatment of Grade 2 astrocytoma or oligodendroglioma with a susceptible IDH1 or IDH2 mutation following surgery. Outcomes of glioma treatments are still unsatisfactory. For example, median survival of glioblastoma patients is approximately 14.6 months, with fewer than 10% of patients surviving 5 years and only 2-3% of patients surviving 10 years beyond diagnosis, representing a very dismal treatment outcome (Weller et al, 2021; Qi et al, 2022; Chen and Cui, 2024). Medical treatments are frequently associated with partially serious adverse events (Chen and Cui, 2024).

Berdel et al. reported that edelfosine exhibited cytostatic effects in a variety of rat and human brain cancer cell lines, including cells freshly taken from patient astrocytomas, glioblastomas, meningiomas, and medulloblastomas (Berdel et al, 1983a; Berdel, 1983b; Berdel 1983c, Berens, 1988; Estella-Hermoso de Mendoza et al, 2011). Another study showed synergistic activity of edelfosine in combination with vincristine against human glioma cell lines D37MG and GaMG (Haugland et al, 1999).

Oral (25 mg/kg on five consecutive days over three weeks) and intrathecal (i.t.) administration of edelfosine (6.25 to 50 mg/kg three times weekly over 21 days) led to a pronounced tumor reduction in male NMRI nu/nu mice transplanted with T 406 human glioblastoma cells. The oral and i.t application of 25 mg/kg edelfosine resulted in growth inhibitions of 78% and 74%, respectively (Zeller et al, 1990).

In the context of a large phase II study conducted with edelfosine (average daily dose: 300 mg) in approx. 1,000 patients with various solid and liquid tumors, in total 41 mostly elderly patients with different types of mostly untreatable progressive and/or recurrent brain tumors (including astrocytomas, glioblastomas, oligoastrocytomas/dendrogliomas) were treated.

After 2 months of treatment, about 56% of the patients showed stable disease (SD) assessed by way of imaging. Another 14% experienced SD based on clinical assessment. In about 24% of all treated patients, the tumor was still progressive after 2 months of therapy with edelfosine. Tolerability was good. The main adverse events included nonserious, easily treatable and reversible gastrointestinal symptoms (e.g., nausea, diarrhea). These data were included in an U.S. patent (Nagler, 2003).

A drug addressing gliomas must be able to pass the blood-brain barrier (BBB). In various publications it was shown that edelfosine was able to penetrate the BBB in rats (Arnold et al, 1978; Unger et al, 1985; Marschner et al, 1992) as well as in healthy and immunocompromised SCID mice (Estella-Hermoso de Mendoza et al, 2009).

During the last decades, enormous progress has been made to successfully treat many types of cancer. However, gliomas still represent malignancies for which up to now no satisfactory treatment options exist. The chemotherapeutic drug temozolomide, still being the basis for treating most gliomas, was approved in 1999, and the VEGF antibody bevacizumab in 2009, respectively. Carmustine and lomustine were introduced in the 1970s, and vincristine in the 1960s. Huge efforts have been made to develop new treatments (e.g., various types of targeted therapies, including different kinase inhibitors, immune-oncology drugs, other monoclonal antibodies, cell therapies including CAR-T cells), but have not yet resulted in new drug approvals to treat gliomas. The only exception was the approval of the IDH inhibitor vorasidenib in 2024.

Correspondingly, outcomes and prognosis as well as quality of life of patients with glioma are still dismal. Survival is poorest in patients with glioblastoma, where median overall survival (OS) was reported to range from 9 to 24 months. Survival at 1 year varied between 29% and 54%, and at 2 years between 10% and 27%. In astrocytoma, median OS was reported to vary between 5.8 months in elderly patients to 23.8 months in diffuse grade 4 (IDH1/2 wildtype astrocytoma with molecular features of glioblastoma) and to 5.2 years for low-grade astrocytoma. Compared with glioblastoma and astrocytoma, oligodendroglioma is associated with better survival. Median OS ranged from 22.6 months in elderly patients to 4.6 years in anaplastic oligodendroglioma treated with PCV and radiotherapy, and to 7.2 years in grade 2 disease (Pöhlmann et al, 2024).

Costs to treat gliomas (in particular advanced stages) and Quality of Life (QoL) for patients with gliomas is often poor (Pöhlmann et al, 2024).

A major limitation for many compounds to be effective anti-glioma treatments is that they could not penetrate the blood-brain barrier (BBB). It was shown in preclinical models that S-CP201 can pass the BBB.

It has therefore been the object of the present invention to provide new treatments for gliomas.

The present invention provides edelfosine (especially (S)-edelfosine, S-CP201) for use in the treatment of gliomas.

The present invention moreover provides edelfosine (especially (S)-edelfosine, S-CP201) for use in the treatment of astrocytomas, ependymomas, glioblastomas multiforme (including IDH-wildtype and IDH-mutant) and/or oligodendrogliomas.

The present invention further provides edelfosine (especially (S)-edelfosine, S-CP201) for use in the treatment of frontline and advanced stages of gliomas.

The present invention moreover provides a combination of edelfosine (especially (S)-edelfosine, S-CP201) with chemotherapeutic agents, such as e.g., temozolomide, lomustine, carmustine, procarbacine, vincristine, or nitrosurea for use in the treatment of gliomas.

The present invention further provides a combination of edelfosine (especially (S)-edelfosine, S-CP201) with the PVC regimen (procarbazine, lomustine, and vincristine) for use in the treatment of gliomas.

The present invention further provides a combination of edelfosine (especially (S)-edelfosine, S-CP201) with bevacizumab for use in the treatment of gliomas.

The present invention further provides a combination of edelfosine (especially (S)-edelfosine, S-CP201) with IDH2 inhibitors (e.g., vorasidenib citrate) for use in the treatment of gliomas.

The present invention further provides edelfosine (especially (S)-edelfosine, S-CP201) for use in the treatment of gliomas in combination with radiotherapy/irradiation.

The present invention moreover provides edelfosine (especially (S)-edelfosine, S-CP201) for use in neo-adjuvant or adjuvant treatment of gliomas.

As stated above, the present invention provides edelfosine for use in the treatment of gliomas.

Preferably, the edelfosine for use in the present invention is (S)-edelfosine (or S-CP201)

Further preferably, the gliomas that are treated according to the present invention are selected from astrocytomas, ependymomas, glioblastomas multiforme (including IDH-wildtype and IDH-mutant) and oligodendrogliomas.

Moreover preferably, the gliomas that are treated according to the present invention are selected from frontline and advanced stages of gliomas.

Further preferably, edelfosine is used in combination with chemotherapeutic agents, such as e.g., temozolomide, lomustine, carmustine, procarbacine, vincristine, and/or nitrosurea.

Moreover preferably, edelfosine is used in combination with the PVC regimen (procarbazine, lomustine, and vincristine).

Further preferably, edelfosine is used in combination with bevacizumab.

Moreover preferably, edelfosine is used in combination with IDH2 inhibitors (e.g., vorasidenib citrate).

Further preferably, edelfosine is used in combination with radiotherapy/irradiation.

Moreover preferably, the treatment of gliomas is neo-adjuvant or adjuvant treatment of gliomas.

It is further preferred to combine the preferred embodiments of the present invention in any desired manner.

The present invention further provides pharmaceutical compositions comprising edelfosine or a pharmaceutically acceptable salt thereof, optionally in combination with one or more carrier substances and/or one or more adjuvants.

In general, the compounds and pharmaceutical compositions described herein will be administered by using the known and acceptable modes known in the art.

The compounds and pharmaceutical compositions of the present invention can be administered by one of the following routes:
- oral, e.g. as tablets (including extended-release), mini tablets, sublingual tablets, buccal tablets, dragees, coated tablets, pills, semisolids, capsules (including extended-release), soft or hard capsules, for example soft and hard gelatine capsules, aqueous or oily solutions, emulsions, suspensions, or syrups, juices or drops (e.g., for children);
- parenteral including intravenous, intramuscular and subcutaneous injection, e.g. as an injectable solution or suspension, pre-filled syringes, or infusion solution concentrates (vials);
- rectal as suppositories;
- by inhalation;
- transdermal or topical, for example via a transdermal delivery system (TDS) such as a plaster containing the active ingredient, or as ointments, creams, or lotions;
- intracranial application (e.g. as a coated wafer for implantation into the brain).

For the production of such tablets, mini tablets, sublingual tablets, buccal tablets, pills, semisolids, coated tablets, dragees and hard, e.g. gelatine, capsules the therapeutically useful product may be mixed with pharmaceutically inert, inorganic or organic excipients as are e.g. lactose, sucrose, glucose, gelatine, malt, silica gel, starch or derivatives thereof, talc, stearinic acid or their salts, dried skim milk, magnesium-alumininum silicates (e.g. Neusilin), 6-O-a-isomaltulose (Isomalt), cross linked polyvinyl N-pyrrolidone (PVP, Crosspovidone), magnesium stearate and the like. For the production of soft capsules, one may use excipients as are e.g. vegetable, petroleum, animal or synthetic oils, wax, fat, and polyols. For the production of liquid solutions, emulsions or suspensions or syrups one may use as excipients e.g. water, alcohols, aqueous saline, aqueous dextrose, polyols, glycerin, lipids, phospholipids, cyclodextrins, vegetable, petroleum, animal or synthetic oils. Especially preferred are lipids and more preferred are phospholipids (preferred of natural origin; especially preferred with a particle size between 300 to 350 nm) preferred in phosphate buffered saline (pH = 7 to 8, preferred 7.4). For suppositories one may use excipients as are e.g. vegetable, petroleum, animal or synthetic oils, wax, fat and polyols. The pharmaceutically useful agents may also contain additives for conservation, stabilization, e.g. UV stabilizers, emulsifiers, sweetener, aromatizers, salts to change the osmotic pressure, buffers, coating additives and antioxidants. If the pharmaceutical compositions are intended to be administered via the oral route to children, it is preferred that they are formulated as a syrup, a sublingual tablet or a fastdissolving tablet. Preferably, formulations for oral administration to children have a pleasant taste.

### Examples

### 1. In vitro data on edelfosine concerning gliomas

The antiproliferative effect of edelfosine on six brain cancer lines (LN-18 [glioblastoma/glioma], LN-229 [glioblastoma], T98G [glioblastoma], U-87MG [glioblastoma/astrocytoma], A172 [glioblastoma], and U-118MG [glioblastoma/astrocytoma]) was investigated, both as single agent and in combination temozolomide, doxorubicin, camptothecin, and erlotinib. For these six cell lines, single agent edelfosine showed IC₅₀s between <1 and 5 µM. A clear synergy between edelfosine and temozolomide in cell lines U-87MG, A172 and U-118MG was noted (average IC₅₀ of combination 4.2 times higher as for edelfosine alone).

### 2. In vitro data on S-CP201 concerning glioma cell lines

In the context of an adopted broad screen against multiple cancer cell lines, the anti-neoplastic activity of S-CP201 was tested in vitro in four human glioma cancer cell lines. The results are shown in Table 1.

**Table 1 shows cytotoxic activity of S-CP201 on four human glioma cell lines.**

| Cell line | SF268 (astrocytoma) | SF295 (glioblastoma) | SNB75 (glioblastoma) | U87MG (glioblastoma) |
|---|---|---|---|---|
| GI₅₀ (µM) | 9.16 | 5.61 | 8.57 | 2.96 |
| GI₅₀: concentration for 50% of maximal inhibition of cell proliferation, should be used for cytostatic (as opposed to cytotoxic) agents. | | | | |
| Cells were incubated for 72 hours, with the highest test concentration of 100 µM. | | | | |

In a further in vitro experiment, the anti-neoplastic activity of S-CP201 on human glioblastoma cell lines SF295, SF268, and SNB75 was evaluated. The Gl₅₀ values were 6.0 µM, 9.3 µM, and 9.5 µM, respectively.

### 3. In vivo data on S-CP201 concerning glioma cell lines

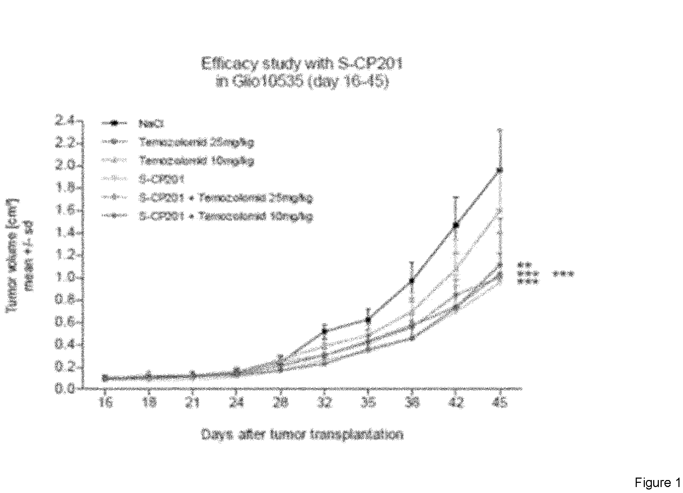

An in vivo efficacy study using patient-derived (PDX) glioblastoma cells was conducted to compare the anti-tumor activity of oral S-CP201, oral temozolomide and the combination S-CP201/temozolomide. Glio10535 cells (glioblastoma WHO grad IV model) were transplanted into female NMRI nude mice. Six groups of six mice each were treated: S-CP201 (10 mg/kg); temozolomide (10 mg/kg); temozolomide 25 mg/kg); S-CP201 (10 mg/kg) + temozolomide (10 mg/kg); S-CP201 (10 mg/kg + temozolomide 25 mg/kg); and control. Treatment commenced on Day 16 post tumor cell inoculation, and was continued until Day 45 post inoculation. The outcomes of the study are displayed in Figure 1.

Figure 1 shows in vivo activity of S-CP201 alone, temozolomide alone and combination of both in the Glio10535 PDX model in mice.

In this in vivo PDX model, S-CP201 significantly decreased the tumor volume in the three treatment arms in which it was included (one as single agent and in two arms two combined with temozolomide). Also, the comparator drug temozolomide (25mg/kg) reduced the tumor volume significantly.

According to RECIST criteria, all groups showed progressive disease (PD) at study end. Overall, S-CP201 (as monotherapy and in the two combination groups with 10 mg/kg and 25 mg/kg temozolomide) was well tolerated.

In conclusion, S-CP201 showed evidence of efficacy in a human PDX glioblastoma model. The drug could be safely combined with the standard of care drug temozolomide.

### 4. Data on blood-brain barrier penetration for S-CP201

A pilot experiment was performed with S-CP201 to investigate its ability to migrate through the BBB in rats. Following single i.v. (2 mg/kg) or oral dosing (10 mg/kg) of S-CP201, the brain penetration of S-CP201 was confirmed in male CD rats (nine animals per group). Plasma and brain sample were taken up to 16 hours post dosing. The brain to plasma ratio was calculated. The brain uptake of S-CP201 was dependent on the route of administration and the systemic plasma levels of S-CP201. The data unequivocally supports the potential utility of S-CP201 for brain tumors. The plasma levels and concentrations detected in the brain are shown in Table 2.

**Table 2: Plasma and brain concentrations of S-CP201 following single-dose application (i.v. and p.o.) in male CD rats**

| | | | | | |
|---|---|---|---|---|---|
| **Intravenous (2 mg/kg)** | **Time (h)** | **Concentration*** | | | **Ratio (Brain to Plasma)** |
| | | **Brain (µg/g)** | **Plasma (ng/mL)** | | |
| | 1 | 0.823 | 2543.333 | | 0.324 |
| | 4 | 0.756 | 1323.333 | | 0.571 |
| | 16 | 0.604 | 481.667 | | 1.125 |
| | | | | | |
| **Oral (10 mg/kg)** | 1 | 0.110 | 84.767 | | 1.298 |
| | 4 | 0.274 | 728.333 | | 0.376 |
| | 16 | 0.891 | 1523.333 | | 0.585 |

In addition, a study was performed to profile the pharmacokinetics (PK) of S-CP201 in mice whole bodies by using MALDI imaging. Oral S-CP201 (20 mg/kg) was given over five days. Following administration of the last dose, mice were sacrificed at distinct time points post last dosing (8 h, 24 h, 72 h, 120 h). The accumulation of S-CP201 was determined in selected organs including brain and in plasma by MALDI-FTICR. Cmax (72.2 µg/mL) in the brain was reached at 72 h, with an AUC0-t of 10.657 µg/mL*h.

### Literature

Arnold B, Reuther R, Weltzien HU: Distribution and metabolism of synthetic alkyl analogs of lysophsphatidylcholine in mice. Biochimica et Biophysica Acta 530: 47-55, 1978 Berdel WE, Fromm M, Fink U, Pahlke W, Bicker U, Reichert A, Rastetter J: Cytotoxicity of thioether-lysophospholipids in leukemias and tumors of human origin. Cancer Res 43: 5538-5543, 1983a
Berdel WE, Greiner E, Fink U, Stavrou D, Reichert A, Rastetter J, Hoffman DR, Snyder F: Cytotoxicity of alkyl-lysophospholipid derivatives and low-alkyl cleavage enzyme activities in rat brain tumor cells. Cancer Res 43: 541-545, 1983b.
Berdel WE, Greiner E, Fink U, Zänker KS, Reichert A, Rastetter J: Cytotoxic effects of alkyl-lysophospholipids in human brain tumor cells. Abstract 1258. Proceedings of the American Associa-tion of Cancer Research 24: 318, 1983c.
Berens ME, Bar-Shira E, Rosenblum ML, Noseda A, Modest EJ: Brain tumor cell sensitivity to ether lipid analogs in vitro. Abstract 1280. Proceedings of the American Association of Cancer Research 29: 322, 1988.
Chen X, Cui Y Zou L: Treatment advances in high-grade gliomas. Front. Oncol., Sec. Neuro-Oncology and Neurosurgical Oncology 14: 1-19, 2024.
Estella-Hermoso de Mendoza A, Campanero MA, de la Iglesia-Vicente J, Gajate C, Mollinedo F, Blanco-Prieto MJ. Antitumor alkyl ether lipid edelfosine: tissue distribution and pharmacokinetic behavior in healthy and tumor-bearing immunosuppressed mice. Clin Cancer Res 15(3): 858-864, 2009.
Estella-Hermoso de Mendoza A, Préat V, Mollinedo F, Blanco-Prieto MJ: In vitro and in vivo efficacy of edelfosine-loaded lipid nanoparticles against glioma. J Control Release 156 (3): 421-6, 2011.
Haugland HK, Nygaard SJ, Tysnes OB: Combined effect of alkyl-lysophospholipid and vincristine on proliferation, migration and invasion in human glioma cell lines in vitro. Anticancer Res 19(1A): 149-56, 1999.
Marschner N, Kötting J, Eibl HJ, Unger C: Distribution of hexadecylphosphocholine and octadecyl-methyl-glycero-3-phosphocholine in rat tissues during steady-state treatment. Cancer Chemother Pharmacol 31: 18- 22, 1992.
Mesfin FB, Karsonovich T, Al-Dhahir MA: Gliomas. StatPearls [Internet], 2025; updated 12 August 2024; https://www.ncbi.nlm.nih.gov/books/NBK441874/.
Nagler A: Edelfosine for the treatment of brain tumors. US Patent No.: US 6,51S,519 B1; 04 February 2003.
Pöhlmann J, Weller M, Marcellusi A, Grabe-Heyne K, Krott-Coi L, Rabar S, Pollock RF: 1High costs, low quality of life, reduced survival, and room for improving treatment: an analysis of burden and unmet needs in glioma. Front. Oncol. Sec. Neuro-Oncology and Neurosurgical Oncology 14: 1-22, 2024
Qi D, Li J, Quarles C, Fonkem E, Wu E: Assessment and prediction of glioblastoma therapy response: challenges and opportunities. Brain 146 (4): 1281-1298, 2022 Schaff LR, Mellinghoff IK: Glioblastoma and other primary brain malignancies in adults. A review. JAMA 329(7): 574-587, 2024.
Unger C. Eibl HJ, von Heyden HW, Krisch B, Nagel GA: Blut-Hirnschranke und Penetration von Zytos-tatika. Klin Wochenschr 63: 565-571, 1985.
Zeller WJ, Bauer S, Remmele T, Wowra B, Sturm V, Stricker H: Interstitial chemotherapy of experi-mental gliomas. Cancer Treatment Reviews 17: 183-189, 1990.
Weller M, van den Bent M, Preusser M, Le Rhun E, Tonn JC, Minniti G, et al: EANO guidelines on the diagnosis and treatment of diffuse gliomas of adulthood. Nature Reviews - Clinical Oncology 18: 170-186, 2021.

## Claims

1. Edelfosine for use in the treatment of gliomas.

2. Edelfosine for use according to claim 1, wherein the edelfosine is (S)-edelfosine.

3. Edelfosine for use according to claim 1 or 2, wherein the gliomas are selected from astrocytomas, ependymomas, glioblastomas multiforme (including IDH-wildtype and IDH-mutant) and oligodendrogliomas.

4. Edelfosine for use according to any one of the preceding claims, wherein the gliomas are selected from frontline and advanced stages of gliomas.

5. Edelfosine for use according to any one of the preceding claims, wherein edelfosine is used in combination with chemotherapeutic agents, such as e.g., temozolomide, lomustine, carmustine, procarbacine, vincristine, and/or nitrosurea.

6. Edelfosine for use according to any one of the preceding claims, wherein edelfosine is used in combination with the PVC regimen (procarbazine, lomustine, and vincristine).

7. Edelfosine for use according to any one of the preceding claims, wherein edelfosine is used in combination with bevacizumab.

8. Edelfosine for use according to any one of the preceding claims, wherein edelfosine is used in combination with an IDH2 inhibitor (e.g., vorasidenib citrate).

9. Edelfosine for use according to any one of the preceding claims, wherein the treatment of gliomas is neo-adjuvant or adjuvant treatment of gliomas.

10. Edelfosine for use according to any one of the preceding claims, wherein edelfosine is used in combination with radiotherapy and/or irradiation.
